# EUROPEAN PATENT APPLICATION

(11) **EP 3 932 296 A1**
(43) Date of publication of application: **05.01.2022**
(21) Application number: 20183389.4
(22) Date of filing: 01.07.2020
(51) Int. Cl.: A61B 5/00, G16H 20/30

(54) **SYSTEM FOR SLEEP RESTRICTION THERAPY**

(71) Applicant: Koninklijke Philips N.V., 5656 AG Eindhoven (NL)
(72) Inventor: WEYSEN, Tim, 5656 AE Eindhoven (NL); LEUFKENS, Timmy Robertus Maria, 5656 AE Eindhoven (NL); MENA BENITO, Maria Estrella, 5656 AE Eindhoven (NL)
(74) Representative: Philips Intellectual Property & Standards

(57) **Abstract**

A sleep restriction therapy system uses historical sleep data of a subject (e.g. based on self-reporting by the subject) to derive a target in-bed duration and additionally a target timing for the target in-bed duration. The timing aims to achieve the most sleep during the in-bed duration. A sleep restriction recommendation is then output based on the target in-bed duration and the target timing.

## Description

### FIELD OF THE INVENTION

This invention relates to sleep restriction therapy, and in particular a system for automating the decision making in sleep restriction therapy.

### BACKGROUND OF THE INVENTION

Insomnia is defined as the perception or complaint of inadequate or poor-quality sleep due to a number of factors, such as difficulty falling asleep, waking up frequently during the night with difficulty returning to sleep, waking up too early in the morning, or unrefreshing sleep. It is one of the most prevalent sleep disorders in the world, since it is generally believed that 10% to 15% of the adult population suffers from chronic insomnia, and an additional 25% to 35% have transient or occasional insomnia.

Traditionally, insomnia has been treated using pharmacologic therapies. During the last decades, non-pharmacologic behavioral therapies have been shown to be effective for the treatment of insomnia. They have many advantages over pharmacologic therapies, including no risk of tolerance, dependence or side effects and also they work on correcting core behavior instead of treating the associated symptoms. Examples of behavioral therapies include: stimulus control therapy, sleep restriction therapy and relaxation training.

Sleep restriction therapy is a very effective behavioral treatment for insomnia that works to decrease variability in the timing of sleep while increasing the depth of sleep. The goal is to shorten the amount of time spent in bed in order to consolidate sleep. It takes however several weeks of diligent dedication to altering a subject's sleep schedule in order to observe improvements in sleep. Subjects feel sleepier and experience more disrupted sleep initially, but the eventual beneficial effects are long lasting.

Sleep Restriction therapy generally incorporates the following steps:
Step 1: Determine an allowed time in bed. This begins by allowing staying in bed for only the average amount of time a subject (i.e. a sleep therapy patient) is actually currently sleeping. This can be calculated by keeping a sleep log for a time period, such as two weeks. The average total sleep time for the monitoring period is determined and 30 minutes is added to derive an allowed time in bed.
Step 2: Set a wake time. The wake up time is set at the same time every morning no matter how much sleep the subject had the night before.
Step 3: Set a go to bed time. The go to bed time is determined by counting back from a desired wake time up by the allowed time in bed set in Step 1. The subject should not get into bed before the go to bed time, even if the subject is sleepy and believes they could fall asleep.
Step 4: Stick to the sleep schedule as closely as possible for at least two weeks. If the subject is sleeping relatively well for most nights and feels good during the day, the sleep schedule is maintained. If the subject feels tired during the day, another 15 minutes is added to the time in bed. The time in bed may for example be increased by 15 minutes per week until the subject is sleeping better at night and feels good during the day.

Other measures should also be taken, such as using a bright light in the morning and dim lights in the evening. Bright light is the most powerful controller of the sleep wake cycle. Using light will help retune a normal sleep/wake pattern, and trying a sleep restriction therapy without a bright light is not nearly as successful. For example, using light for 30 minutes upon awakening is sufficient to regulate the sleep/wake cycle. The subject should avoid napping (as this will decrease night-time sleep drive).

An issue with the current approach arises in particular in Step 3. The go to bed time calculated can be quite late for subjects, and much later than their habitual time to go to bed. This is especially when the calculated Average Total Sleep Time in Step 1 is less than would be desired by the subject. As a result, subjects might resist this sleep window and as such are less motivated to adhere to the new bedtime schedule.

There is therefore a need for an improved way to implement a sleep restriction therapy.

### SUMMARY OF THE INVENTION

The invention is defined by the claims.

According to examples in accordance with an aspect of the invention, there is provided a processor for a system for sleep restriction therapy, comprising:
an input for receiving sleep data for a subject for a plurality of nightly sleep sessions, which indicates at least time epochs during a sleep session that the subject is asleep and time epochs during the sleep session that the subject is awake,
wherein the processor is adapted to process the sleep data to:
   derive from the sleep data a target in-bed duration;
   derive from the sleep data a target timing for the target in-bed duration which timing is selected to achieve the most sleep during the in-bed duration; and
   output a sleep restriction recommendation based on the target in-bed duration and the target timing.

The output may be intended for the subject undergoing the therapy, or for a clinician, or both.

This processor receives sleep data for a plurality of sleep sessions. One sleep session is one period of intended sleep, i.e. one night. The actual sleep performance of the subject is analyzed over a sequence of sleep sessions (i.e. nights) and from this analysis a sleep restriction recommendation is generated.

The sleep data is for example based on self-reporting by the subject, for example by indicating for a sequence of epochs during the night whether they were asleep or awake.

The sleep restriction recommendation comprises a go to bed time and a get up time. Thus, it defines both the duration of the allowed time in bed, but also the best timing. In this way, rather than setting the time in bed e.g. as immediately preceding a desired time of the subject to get up, the time window is set in order to achieve the best sleep results for that subject. This is based on the sleep pattern of the subject, i.e. when they are awake and when they are asleep during the night. In this way, the system improves the success rate of the sleep restriction therapy.

The processor thus provides automatic determination of the so-called consolidated sleep window, and thereby provides a way to optimize the sleep restriction therapy for a subject. The invention may for example enable sleep care professionals to increase the success of the therapy.

Each epoch is for example a period with a duration of 15 minutes. This is the most common time interval for self reporting in the case of sleep restriction therapy. However, shorter epochs (5 minutes, or 10 minutes) or longer epochs (30 minutes, 45 minutes, 1 hour) may be used.

The sleep restriction recommendation may be used to control a system which provides a bed time indicator and a wake up time indicator. Such a system may be a basic alarm or it may be a more elaborate sleep control system (e.g. which controls the lighting and/or sound at bed time and controls the lighting and/or sound at wake up time).

The processor may for example be part of a mobile phone or tablet on which a suitable app has been loaded. The processor may be part of an output device by which the sleep restriction recommendation is provided to the subject or clinician, or it may be a different device.

The processor may derive the target timing by determining the number of occurrences of actual sleep during the time epochs of the plurality of sleep sessions.

Thus each time epoch is assessed to determine if the subject was asleep (for all of the time epoch) or had a period of wakefulness within the time epoch.

The processor may then be further adapted to derive the target timing by determining the probability of achieving sleep in each time epoch based on the occurrences of actual sleep during the time epochs of the plurality of sleep sessions.

Thus, each time epoch is associated with a probability of the subject being able to sleep at that particular portion of the sleep session (i.e. at that time of night). The in-bed duration can then be positioned with respect to time to maximize the total probability.

The input may be further for receiving sleep preferences of the subject, wherein the processor is adapted to derive the target timing taking into account the sleep preferences.

The sleep preferences for example may relate to latest times the subject can stay in bed (e.g. because of work commitments), so that the wake up time has a latest possible time point.

The plurality of sleep sessions for example comprises between 7 and 21 sleep sessions. Thus, the sleep pattern may be analyzed for one to three weeks before a sleep restriction recommendation is generated.

The processor may derive the target in-bed duration based on an estimate of the average total actual sleep time for the plurality of sleep sessions.

The allocated time in bed thus corresponds to the actual amount of sleep the subject generally achieves. This is the basic operation of a sleep restriction therapy approach.

The go to bed time may be set never to be earlier than the actual fall asleep time which has been observed historically. Thus the subject should be ready for sleep when they go to bed.

The processor may be further adapted to modify the sleep restriction recommendation over time based on new sleep data received over time.

Thus, the progression over time of a sleep restriction therapy may also be handled by the system based on the continuously collected sleep data.

The sleep data for example comprises subject input. Sleep questionnaires/diaries can be used to provide at least the initial the sleep data. Thus, self-reporting of the sleep performance during a preceding night may be used as the primary source of data. This typically the case for sleep restriction therapy. The probable over-exaggeration of lack of sleep which is typical in self-reported sleep data is factored into the analysis of the data.

The sleep data may optionally also comprise sensor data. Sensor data may allow more accurate data than self reporting, for example for periods in the middle of a sleep session. It may also allow shorter time epochs to be used.

The invention also provides a system for sleep restriction therapy, comprising:
a sensor arrangement for collecting sensor data; and
the processor defined above for processing the sensor data to generate the sleep restriction recommendation.

In this way, a system includes sensors to supplement the self-reporting data.

The sensor arrangement may comprise a single sensor unit or a system of sensor units. The sensor arrangement may comprise at least one body-worn sleep sensor. This may be a head band or a wrist band. A head band for example enables EEG monitoring, and such sleep tracking headband devices are well known. PPG and motion monitoring using a wrist watch type device is also well known for providing sleep data.

The sensor arrangement may instead or additionally comprise at least one monitoring sensor remote from the subject. This may for example monitor movements, but also provide additional environmental information of interest. This sensor arrangement may for example report times when lights are turned on or off, or provide temperature monitoring (to sense the presence of a body) etc. These may provide additional data for verifying or enhancing the self reporting data or data from body-worn sensors.

The invention also provides a computer-implemented method of generating a sleep restriction recommendation for sleep restriction therapy, comprising:
receiving sleep data for a subject for a plurality of nightly sleep sessions, which indicates at least time epochs during a sleep session that the subject is asleep and time epochs during the sleep session that the subject is awake;
deriving from the sleep data a target in-bed duration;
deriving from the sleep data a target timing for the target in-bed duration which timing is selected to achieving the most sleep during the in-bed duration; and
outputting the sleep restriction recommendation based on the target in-bed duration and the target timing.

The method may comprises:
deriving the target timing by determining the number of occurrences of actual sleep during the time epochs of the plurality of sleep sessions; and
deriving the target in-bed duration based on an estimate of the average total actual sleep time for the plurality of sleep sessions.

The method may further comprise adapting the sleep restriction recommendation over time based on new sleep data received over time.

The invention also provides a computer program comprising computer program code which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a system for sleep restriction therapy;
Figure 2 shows the method implemented by the processor of Figure 1;
Figure 3 shows a dashboard of a subject report logging system;
Figure 4 shows a key for the symbols in the dashboard of Figure 3;
Figure 5 shows a summary which may be prepared in respect of the two week sleep reporting period;
Figure 6 is used to show how the sleep restriction recommendation is determined in accordance with the invention; and
Figure 7 shows an example of a sleep restriction recommendation provided to the subject.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a sleep restriction therapy system in which historical sleep data of a subject (e.g. based on self-reporting by the subject) is used to derive a target in-bed duration and additionally a target timing for the target in-bed duration. The timing aims to achieve the most sleep during the in-bed duration. A sleep restriction recommendation is then output based on the target in-bed duration and the target timing.

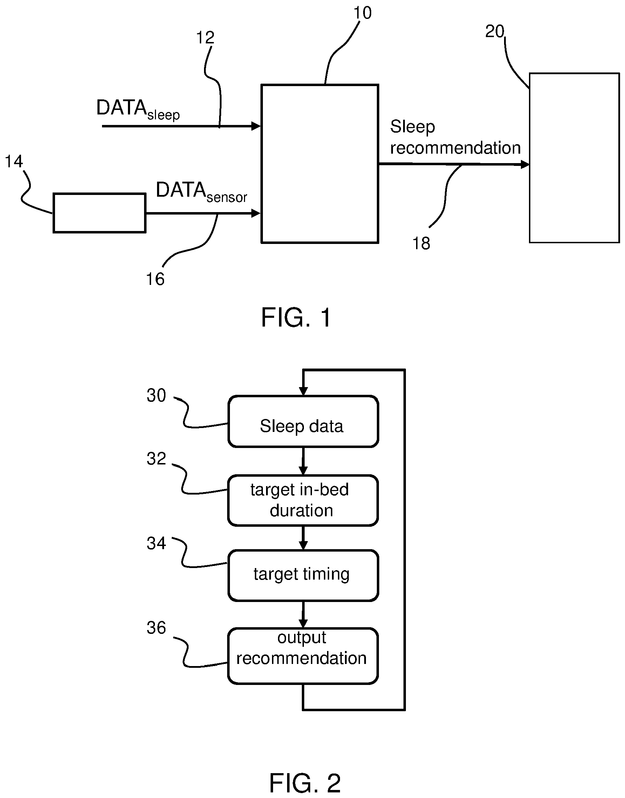

Figure 1 shows a system for sleep restriction therapy, comprising a processor 10 and a user interface device 20. The user interface device is a device for providing instructions to the subject, such as an alarm clock which may set both a go to bed time and a wake up time. The user interface device may simply comprise a mobile phone or tablet on which suitable software is loaded, and the processor 10 may then be the processor of that device. Alternatively, the processor may be part of a separate device which then communicates with the user interface device 20.

The processor has an input 12 for receiving sleep data DATAₛₗₑₑₚ for a subject for a plurality of nightly sleep sessions, which indicates at least time epochs during a sleep session that the subject is asleep and time epochs during the sleep session that the subject is awake. The processor includes a memory (database) for storing the data for subsequent analysis.

This sleep data is for example based on a self-reporting sleep report such as a sleep diary. Optionally, a sensor arrangement 14 is provided for collecting sensor data DATAₛₑₙₛₒᵣ. The processor additionally processes the sensor data. The sensor arrangement is for example used to supplement the self-reporting data. The sensor arrangement 14 may comprise at least one body-worn sleep sensor. This may be a head band or a wrist band. A head band for example enables EEG monitoring, and such sleep tracking headband devices are well known. PPG and motion monitoring using a wrist watch type device is also well known for providing data indicating sleep information. Contact-less sensors may also be used for remote sensing.

The processor 10 processes the sleep data to derive from the sleep data (and optionally also the sensor data) a target in-bed duration. It also derives from the sleep data (and optionally also the sensor data) a target timing for the target in-bed duration which timing is selected to achieve the most sleep during the in-bed duration. A sleep restriction recommendation 18 is then generated based on the target in-bed duration and the target timing. This is provided to the user interface device 20.

This processor receives sleep data for a plurality of sleep sessions. One sleep session is one period of intended sleep, i.e. a time in bed during one night. The actual sleep performance of the subject is analyzed over a sequence of sleep sessions (i.e. nights) and from this analysis the sleep restriction recommendation is generated.

The sleep data is for example based on self-reporting by subject, for example by indicating for a sequence of epochs during the night whether they were asleep or awake.

Figure 2 shows the method implemented by the processor.

The method comprises, in step 30, receiving sleep data for a subject for a plurality of nightly sleep sessions, which indicates at least time epochs during a sleep session that the subject is asleep and time epochs during the sleep session that the subject is awake.

In step 32, a target in-bed duration is derived from the sleep data.

In step 34, a target timing for the target in-bed duration is derived. This timing is selected to achieving the most sleep during the in-bed duration. A sleep restriction recommendation is output based on the target in-bed duration and the target timing.

The sleep data input may for example be based on a digital sleep/wake diary maintained by the subject for self-reporting of their sleep/awake timings. The reporting to the diary could be achieved using a user interface to a digital diary, for example by means of a dedicated app on their smartphone or tablet, or an existing app associated with the user interface device 20.

The diary is for example maintained for a period of sleep 1 or 2 weeks prior to the intervention. Using the digital sleep diary, and optionally also a sensor arrangement for sleep tracking, insights into the sleep characteristics prior to the therapy are provided to the processor.

The aim of the sleep restriction recommendation is to select a time window during the night during which the probability of the subject being asleep is the highest.

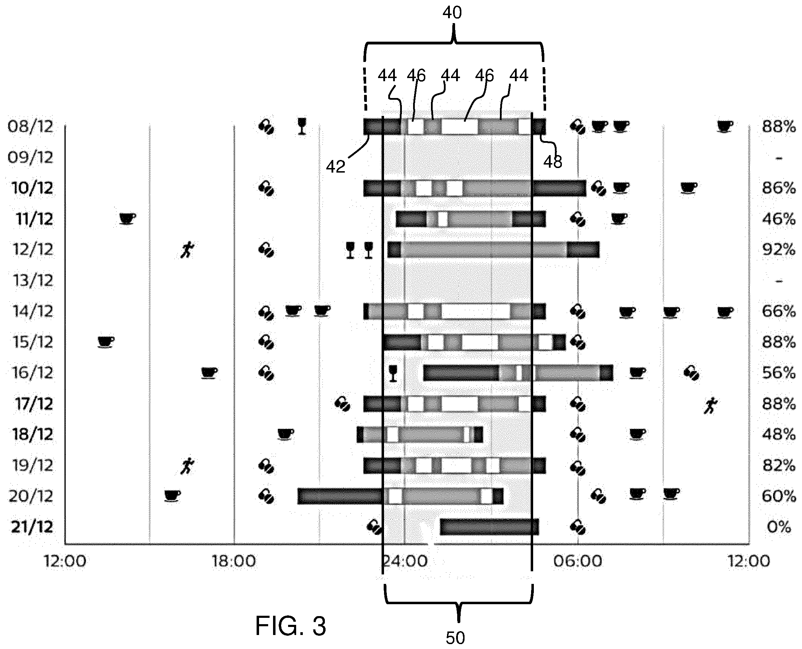

Figure 3 shows the dashboard of a subject sleep report logging system, by which subjective data is collected from a subject during a period of 14 days (the left y-axis shows dates from 8 December to 21 December). Such a logging system may then be adapted to provide the sleep restriction recommendation in accordance with the invention.

For each day, the subject reports various events (such as their exercise and food and drink intake) during awake periods (discussed below) and their sleep report. For example, the time in bed for the first day is shown as period 40. This time period may be considered to be a "sleep session".

The initial part 42 of the sleep session is coded with a first color, to represent the time to fall asleep after going to bed. After this, there are periods 44 of sleep and periods 46 of wakefulness, again coded with different colors. After the final period of sleep, there is a period 48 when the subject is awake in bed before they get up.

Thus, periods when the subject is in bed, out of bed, awake and asleep are indicated. This self-reported data is for example visualized to a clinician. In addition, a consolidated sleep window 50 is identified. This corresponds to the sleep restriction recommendation, and the way it is derived is explained below. The consolidated sleep window 50 is for example a time window of at least 5 hours, and it is selected such that the chance of being asleep is highest.

The start time (the time to go to bed) and the end time (the time to get out of bed) is used by the subject or his clinician as the sleep restriction recommendation for a new bedtime schedule. The recommendation can for example be discussed together with the subject and may be visualized via a daily reminder in a connected smartphone application.

The dashboard of Figure 3 includes additional information which may be of use to a clinician, but not directly relevant to the process of determining the consolidated sleep window.

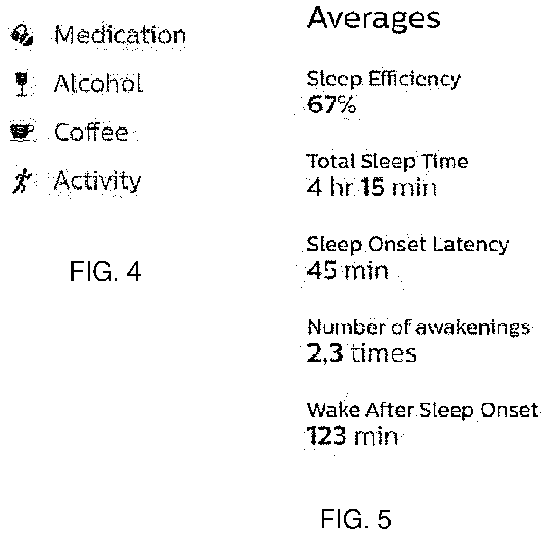

Figure 4 shows a key for the symbols in the dashboard of Figure 3, indicating the timings at which medication, alcohol or coffee were taken and the time when exercise was undertaken.

Figure 5 shows a summary which may be prepared in respect of the two week sleep reporting period, showing the sleep efficiency, average total sleep time, sleep onset latency, number of awakenings and the time to wake after the first onset of sleep. These may all be of relevance to the clinician in providing suitable advice.

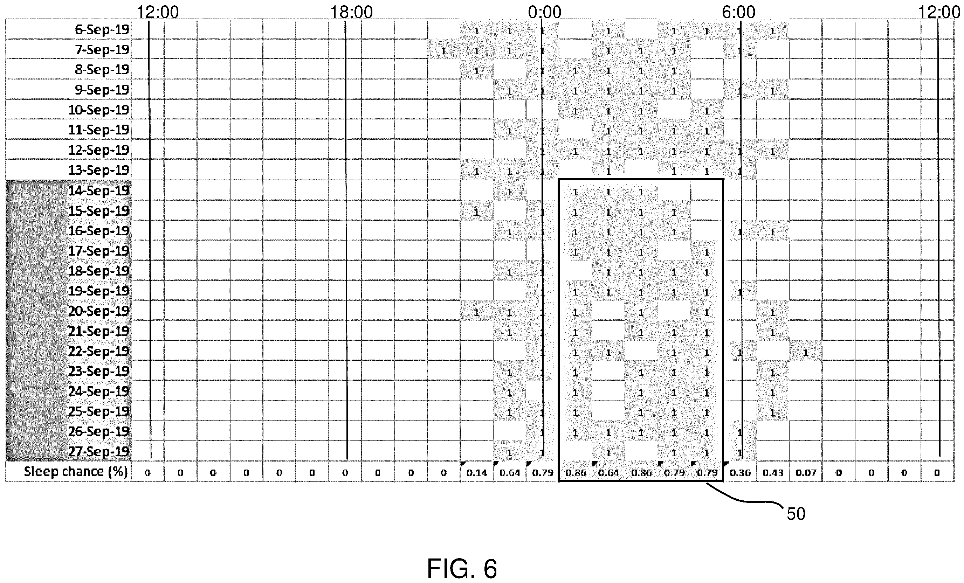

Figure 6 is used to show, in simplified form, how the sleep restriction recommendation is determined.

The sleep data is converted into binary form. Each sleep session is divided into time epochs. These are typically 15 minute time periods. To simplify the image, Figure 6 shows time epochs of 1 hour.

Each time epoch is allocated a binary value. 1 represents a time epoch when the subject was asleep the whole time. 0 represents a time when the subject was awake for at least part of that time epoch. Other coding approaches are of course possible, such as 1 for asleep for at least most of the time, and 0 for awake for at least most of the time.

For every fixed time epoch (60 minutes in this simplified example), the chance of being asleep, looking at the previous monitoring period, e.g. 14 days, is calculated. For example, for the time slot 11:30pm to 12:30pm, the subject was asleep for 11/14 of the previous 14 days, hence a probability of 0.79 as shown in the bottom row.

Thus the processor determines the number of occurrences of actual sleep during the time epochs of the plurality of sleep sessions.

The consolidated sleep window 50, which is the allowed time in bed, has an initial duration of 5 hours. This will typically adapt over time as the therapy advances.

The timing of the 5 hour window, i.e. the target timing of the in-bed duration, can then be positioned within the night to maximize the total probability. Thus, the position in time is selected to maximize the sum of the five consecutive values of the probability values in the last row. In this case, there are two equal positions; 0:30am to 5:30am or 12:30am to 4:30am.

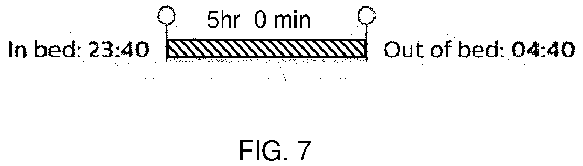

Figure 7 shows an example of a sleep restriction recommendation provided to the subject with a finer 10 minute resolution, based on the data of Figure 3. It shows the go to bed time and the get up time, as well as the duration. This will be provided as part of the dashboard shown in Figure 3.

The processor may also take account of sleep preferences of the subject. For example, a subject may prefer to go to bed early and get up early if the probability of success of the sleep therapy is the same. The sleep preferences for example may relate to latest times the subject can stay in bed (e.g. because of work commitments), or the earliest time the subject can go to bed (e.g. because of evening commitments).

The plurality of sleep sessions does not have to be two weeks. More generally it may be between 7 and 21 sleep sessions. Thus, the sleep pattern may be analyzed for one to three weeks before a sleep restriction therapy recommendation is generated.

The sleep restriction therapy may start with a fixed time in bed window, e.g. of 5 hours as explained above. However, the target in-bed duration may instead be based on an estimate of the average total actual sleep time for the plurality of sleep sessions. Thus, the starting point may be based on the actual amount of sleep the subject generally achieves. This is the basic operation of a sleep restriction therapy approach.

The go to bed time may be set never to be earlier than the actual fall asleep time which has been observed historically. Thus the subject should be ready for sleep when they go to bed.

The sleep restriction recommendation will evolve over time. The processor may thus modify the sleep restriction recommendation over time based on new sleep data received over time. Thus, the progression over time of a sleep restriction therapy may also be handled by the system based on the continuously collected sleep data.

The sensor arrangement mentioned above may be a body-worn sensor, so that the processor may for example be used to extend the functionality of a sleep monitoring headband. The sensor arrangement may instead or additionally comprise at least one monitoring sensor which is contact-less. This may for example monitor movements, but also provide additional environmental information of interest. This sensor arrangement may for example report times when lights are turned on or off, or provide temperature monitoring (to sense the presence of a body) etc. These may provide additional data for verifying or enhancing the self reporting data or data from body-worn sensors.

The processing of the data may be handled in various ways, either locally to the subject, or remotely. For example, data may be stored in a remote database.

The system may for example automatically generate summarizing reports to give the specialist a quick overview of the data. The clinician/doctor can review the data in a graphical interface and use this to discuss the new bedtime schedule with the subject. This may enable easy adaption of treatment to maximize individual adherence.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processor (10) for a system for sleep restriction therapy, comprising:
an input (12) for receiving sleep data (DATAₛₗₑₑₚ) for a subject for a plurality of nightly sleep sessions (40), which indicates at least time epochs (44) during a sleep session that the subject is asleep and time epochs (46) during the sleep session that the subject is awake,
wherein the processor (10) is adapted to process the sleep data to:
derive from the sleep data a target in-bed duration;
derive from the sleep data a target timing for the target in-bed duration which timing is selected to achieve the most sleep during the in-bed duration; and
output a sleep restriction recommendation based on the target in-bed duration and the target timing.

2. The processor of claim 1, further adapted to derive the target timing by determining the number of occurrences of actual sleep during the time epochs of the plurality of sleep sessions.

3. The processor of claim 2, further adapted to derive the target timing by determining the probability of achieving sleep in each time epoch based on the occurrences of actual sleep during the time epochs of the plurality of sleep sessions.

4. The processor of any one of claims 1 to 3, wherein the input is further for receiving sleep preferences of the subject, wherein the processor is adapted to derive the target timing taking into account the sleep preferences.

5. The processor of any one of claims 1 to 4, wherein the plurality of sleep sessions comprises between 7 and 21 sleep sessions.

6. The processor of any one of claims 1 to 5, adapted to derive the target in-bed duration based on an estimate of the average total actual sleep time for the plurality of sleep sessions.

7. The processor of any one of claims 1 to 6, further adapted to modify the sleep restriction recommendation over time based on new sleep data received over time.

8. The processor of any one of claims 1 to 7, wherein the sleep data comprises:
subject input; and
optionally, sensor data.

9. A system for sleep restriction therapy, comprising:
a sensor arrangement (14) for collecting sensor data; and
the processor of any one of claims 1 to 8 for processing the sensor data to generate the sleep restriction recommendation.

10. The system of claim 9, wherein the sensor arrangement comprises at least one body-worn sleep sensor.

11. The system of claim 9 or 10, wherein the sensor arrangement comprises at least one monitoring sensor remote from the subject.

12. A computer-implemented method of generating a sleep restriction recommendation for sleep restriction therapy, comprising:
(30) receiving sleep data for a subject for a plurality of nightly sleep sessions, which indicates at least time epochs during a sleep session that the subject is asleep and time epochs during the sleep session that the subject is awake;
(32) deriving from the sleep data a target in-bed duration;
(34) deriving from the sleep data a target timing for the target in-bed duration which timing is selected to achieving the most sleep during the in-bed duration; and
(36) outputting the sleep restriction recommendation based on the target in-bed duration and the target timing.

13. The method of claim 12, comprising:
deriving the target timing by determining the number of occurrences of actual sleep during the time epochs of the plurality of sleep sessions; and
deriving the target in-bed duration based on an estimate of the average total actual sleep time for the plurality of sleep sessions.

14. The method of any one of claims 12 to 13, further comprising adapting the sleep restriction recommendation over time based on new sleep data received over time.

15. A computer program comprising computer program code which is adapted, when said program is run on a computer, to implement the method of any of claims 12 to 14.
